(19) Europäisches Patentamt
European Patent Office
Office européen des brevets

(11) Numéro de publication: **0 100 717**
**B1**

(12) **FASCICULE DE BREVET EUROPEEN**

(45) Date de publication du fascicule du brevet: **06.11.85**

(51) Int. Cl.⁴: **C 07 C 95/02,** C 07 C 125/065

(21) Numéro de dépôt: **83401519.0**

(22) Date de dépôt: **25.07.83**

(54) **Procédé de synthèse d'alphaalkyl amino aldéhydes.**

(30) Priorité: **30.07.82 FR 8213385**

(43) Date de publication de la demande: **15.02.84 Bulletin 84/7**

(45) Mention de la délivrance du brevet: **06.11.85 Bulletin 85/45**

(84) Etats contractants désignés: **AT BE CH DE FR GB IT LI LU NL SE**

(56) Documents cités:
**SAUL PATAI: "THE CHEMISTRY OF THE CARBONYL GROUP", 1966, pages 221-225, "Formation from carboxylic acids and their derivatives", Interscience Publishers, Londres, GB.**
**J. ORG. CHEM., vol. 46, no. 23, 1981, pages 4797-4798, American Chemical Society, Washington, D.C., USA C. FREEMAN et al.: "Preparation of protected amino aldehydes"**

(73) Titulaire: **SANOFI, société anonyme, 40, Avenue George V, F-75008 Paris (FR)**

(72) Inventeur: **Castro, Bertrand, 7 rue Hélène Boucher, F-34470 Perols (FR)**
Inventeur: **Fehrentz, Jean, 176 rue de Leyde, F-34100 Montpellier (FR)**

(74) Mandataire: **Gillard, Marie-Louise et al, Cabinet Beau de Loménie 55, Rue d'Amsterdam, F-75008 Paris (FR)**

**EP 0 100 717 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La famille des α-alkylaminoaldéhydes présente un grand intérêt lié tant aux propriétés thérapeutiques de ces composés qu'à la possibilité de leur utilisation en tant qu'intermédiaire de synthèse.

Toutefois, la synthèse de tels composés est délicate et, lorsqu'il s'agit de molécules chirales, principalement à cause de la racémisation très facile du carbone asymétrique de la molécule.

Jusqu'à présent la préparation d'α-alkylaminoaldéhydes résultait, soit de la réduction des α-aminoacides ou α-aminoesters correspondants, soit de l'oxydation des alcools correspondants.

Dans tous les cas, les rendements assez faibles nécessitaient des purifications plus ou moins laborieuses et la racémisation des produits demeurait un problème majeur conduisant à des produits de pouvoir rotatoire très faible [voir en particulier «Journal of Organic Chemistry», *46*, 4797, (1981)].

La présente invention a pour objet une nouvelle méthode de préparation d'α-alkylaminoaldéhydes permettant d'éviter les phénomènes de racémisation et conduisant avec un bon rendement à l'obtention d'α-alkylaminoaldéhydes présentant un pouvoir rotatoire élevé.

Ce procédé est résumé dans le schéma réactionnel suivant:

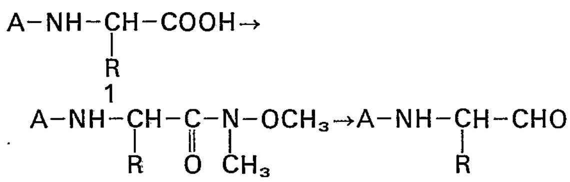

1, 2, 3

où A représente un groupe susceptible de bloquer de façon réversible la fonction amine.

Le produit de départ est l'α-aminoacide

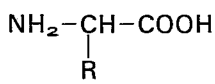

dans lequel R est un groupe alkyle droit ou ramifié, ou un groupe aralkyle pouvant être éventuellement substitué par un ou des groupes hydroxy, thiol ou NH–R', R' étant un atome d'hydrogène ou un radical alkyle. En particulier, R peut représenter la chaîne latérale des α-aminoacides naturels.

Afin d'éviter toute réaction parasite intra- ou intermoléculaire au cours de la préparation des aminoaldéhydes, il est nécessaire au préalable de bloquer la fonction amine. On utilise avantageusement un groupe acyle tel que le groupe t-butoxycarbonyle (Boc).

De même, lorsque la chaîne latérale R comporte des substituants réactifs, il est nécessaire de bloquer ceux-ci au préalable. On peut utiliser les groupes généralement utilisés en synthèse peptidique pour cet usage et en particulier l'éther benzylique.

La première étape de la synthèse consiste à préparer les N-méthoxy N-méthylamides 2 par action de la N,O-diméthylhydroxylamine sur un ester activé de l'aminoacide 1. L'ester activé est préparé *in situ* au sein d'un solvant convenable tel que le chlorure de méthylène ou l'acétate d'éthyle par une méthode connue en soi, puis, sans isolement par addition de N,O-diméthylhydroxylamine en milieu basique, on obtient les produits 2. Les produits 2 se présentent en général comme des huiles; ils sont très stables et peuvent être conservés sans décomposition apparente.

La seconde étape consiste à réduire les N-méthoxy N-méthylamides 2 ainsi obtenus par un hydrure double et de préférence l'hydrure de lithium-aluminium en excès.

On opère à température peu élevée de préférence à 0° C au sein d'un solvant convenable tel que l'éther ou le tétrahydrofuranne.

Après isolement, les α-alkylaminoaldéhydes sont obtenus avec un rendement élevé. Ils se présentent le plus souvent sous forme d'huile de pureté satisfaisante.

Ils présentent un pouvoir rotatoire élevé, contrairement aux produits décrits dans la littérature.

Les exemples suivants, nullement limitatifs, permettent de mieux comprendre l'invention.

Dans ce qui suit, on utilisera pour plus de simplicité les abréviations suivantes:

Leu = Leucine,
Boc = Tertiobutoxycarbonyle.

Les spectres de RMN ont été enregistrés à 250 MHz en solution dans le deutérochloroforme, la référence interne étant le tétraméthylsilane.

Les abréviations suivantes ont été utilisées:

s = singulet,
d = doublet,
t = triplet,
q = quadruplet,
m = massif,

J représente la constante de couplage exprimée en hertz.

*Exemple 1:*

*(Tertiobutoxycarbonylamino)-2 méthyl-4 pentanal*

3 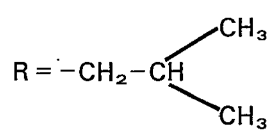

a) 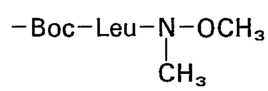

2 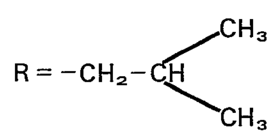

A 2,15 g de L-Boc Leucine dissous dans 50 ml de chlorure de méthylène, on ajoute 1,3 g de diisopropyléthylamine et 4,26 g d'hexafluorophosphate de N-benzotriazolyloxy tris-diméthylaminophosphonium. On ajoute ensuite 1,07 g de chlorhydrate de N,O-diméthylhydroxylamine et 1,42 g de diisopropyléthylamine et laisse sous agitation.

On suit la réaction par chromatographie en couche mince d'un échantillon. Lorsque la réaction est complète (30 à 60 min) on ajoute du chlorure de méthylène et lave la solution successivement avec une solution d'acide chlorhydrique 3N puis avec une solution saturée de bicarbonate de sodium et enfin avec une solution saturée de chlorure de sodium.

On sèche la solution sur sulfate de magnésium et évapore le solvant à siccité sous vide. Le résidu huileux est chromatographié sur silice pour fournir une huile visqueuse incolore.

Rendement 94%.

$\alpha_D^{20}$ = − 22,7° C (c = 1% méthanol).

*Spectre de RMN:*

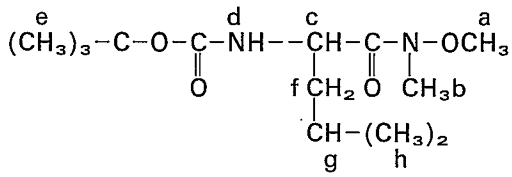

1H à 5,32 ppm ($H_d$, d, $J_{dc}$ = 7,5); 1H à 4,73 ppm ($H_c$, dt, $J_{cd}$ = 7,5); 3H à 3,79 ppm ($H_a$, S); 3H à 3,19 ppm ($H_b$, S); 1H à 1,81-1,65 ppm ($H_g$, m); 2H à 1,52-1,30 ppm ($H_f$, m); 9H à 1,44 ppm ($H_e$, S); 6H à 0,95 ppm ($H_h$, 2d, $J_{hg}$ = 6,5).

b) -Boc-Leu-H

A une solution de 1,1 g de l'amide obtenu ci-dessus dans 40 ml d'éther, on ajoute à 0° C 0,19 g d'hydrure double de lithium-aluminium. On laisse sous agitation pendant 20 min puis on hydrolyse avec une solution aqueuse contenant 0,95 g de sulfate acide de potassium. On ajoute 100 ml d'éther, sépare la phase aqueuse qu'on réextrait avec de l'éther.

On réunit les phases organiques et on les lave successivement avec une solution d'acide chlorhydrique 3N, puis avec une solution saturée de bicarbonate de sodium et enfin avec une solution saturée de chlorure de sodium.

On sèche sur sulfate de magnésium et évapore le solvant à siccité.

On obtient une huile incolore.

Rendement 96%.

$\alpha_D^{20}$ = −57,3° C (c = 1% méthanol).

Ce produit doit être conservé sous atmosphère d'argon.

*Spectre de RMN:*

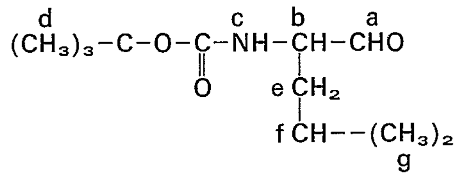

1H à 9,6 ppm ($H_a$, S); 1H à 5,48 ppm ($H_c$, d, $J_{cb}$ = 7); 1H à 4,2 ppm ($H_b$, m); 3H à 1,9-1,45 ppm ($H_e$, $H_f$, m); 9H à 1,45 ppm ($H_d$, s); 6H à 0,96 ppm ($H_g$, d, $J_{gf}$ = 6,5).

*Exemples 2 à 6:*

a) En opérant comme dans l'exemple 1a mais en faisant varier le L-amino protégé utilisé comme produit de départ on obtient les N-méthyl N-méthoxyamides 2 réunis dans le tableau I.

*Tableau I*

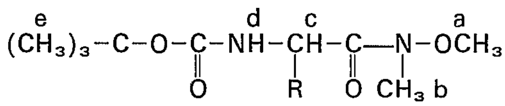

| Exemple N° | R | Rendement (%) | $\alpha_D^{20}$ (1% méthanol) | Point de fusion ° C ou spectre de RMN |
|---|---|---|---|---|
| 2 a | $-CH_3$ | 85 | −26,8 | F=150 |
| 3 a | 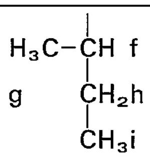 | 70 | −21,2 | 1H à 5,50 ppm ($H_d$, d, $J_{dc}$=9,5); 1H à 4,62 ppm ($H_c$, dd, $J_{cd}$=9,5); 3H à 3,78 ppm ($H_a$, S); 3H à 3,20 ppm ($H_b$, S); 1H à 1,74 ppm ($H_f$, m); 9H à 1,42 ppm ($H_e$, S); 2H à 1,22-1,01 ppm ($H_h$, m); 3H à 0,90 ppm ($H_g$, d, $J_{gf}$=7); 3H à 0,88 ppm ($H_i$, t, $J_{ih}$=7) |
| 4 a | 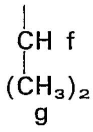 | 80 | −16 | 1H à 5,21 ppm ($H_d$, d, $J_{dc}$=9); 1H à 4,58 ppm ($H_c$, dd, $J_{cd}$=9, $J_{cf}$=7); 3H à 3,78 ppm ($H_a$, S); 3H à 3,22 ppm ($H_b$, S); 1H à 1,99 ppm ($H_f$, m, J=7); 9H à 1,44 ppm ($H_e$, S); 3H à 0,96 ppm ($H_g$, d, $J_{gf}$=7); 3H à 0,91 ppm ($H_g$, d, $J_{gf}$=7) |
| 5 a | 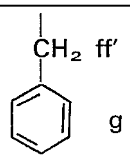 | 95 | + 2,6 | 5H à 7,6 ppm ($H_g$, m); 1H à 5,68 ppm ($H_d$, d, $J_{dc}$=8,5); 1H à 4,95 ppm ($H_c$, ddd, $J_{cd}$=8,5, $J_{cf}$=$J_{cf'}$=6); 3H à 3,60 ppm ($H_a$, S); 3H à 3,10 ppm ($H_b$, S); 1H à 3,05 ppm ($H_f$, dd, $J_{fc}$=6, $J_{ff'}$=13,5); 1H à 2,87 ppm ($H_{f'}$, dd, $J_{f'c}$=6, $J_{f'f}$=13,5); 9H à 1,38 ppm ($H_é$, S) |

| Exemple N° | R | Rendement (%) | $\alpha_D^{20}$ (1% méthanol) | Point de fusion ° C ou spectre de RMN |
|---|---|---|---|---|
| 6 a | 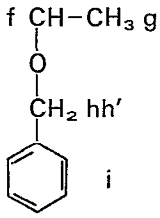 | 95 | −20,8 | 5H à 7,3 ppm ($H_i$, m); 1 H à 5,52 ppm ($H_d$, d, $J_{dc}$=9); 1 H à 4,62 ppm ($H_c$, dd, $J_{cd}$=9, $J_{cf}$=3); 1 H à 4,54 ppm ($H_h$, d, $J_{hh'}$=12); 1 H à 4,40 ppm ($H_{h'}$, d, $J_{hh'}$=12); 1 H à 3,89 ppm ($H_f$, dq, $J_{fc}$=3, $J_{fg}$=6,5); 3H à 3,63 ppm ($H_a$, S); 3H à 3,11 ppm ($H_b$, S); 9H à 1,44 ppm ($H_e$, S); 3H à 1,24 ppm ($H_g$, d, $J_{gf}$=6,5) |

b) A partir des amides figurant dans le tableau I, en opérant comme dans l'exemple 1 b), on obtient les aminoaldéhydes correspondants 3 rassemblés dans le tableau II.

*Tableau II*

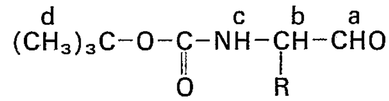

| Exemple N° | R | Rendement (%) | $\alpha_D^{20}$ (1% méthanol) | Point de fusion ° C ou spectre de RMN |
|---|---|---|---|---|
| 2 b | $-CH_3$ (note 1) | 88 | −34,1 | F=88−9 |
| 3 b | e HC $CH_3$<br>$CH_2$ g<br>$CH_3$ h | | | 1H à 9,68 ppm ($H_a$, S); 1H à 5,34 ppm ($H_c$, d, $J_{cb}$=7); 1H à 4,28 ppm ($H_b$, dd, $J_{bc}$=7, $J_{be}$=5); 1H à 2,12-1,92 ppm ($H_e$, m); 9H à 1,42 ppm ($H_d$, S); 2H à 1,20-1,0 ppm ($H_g$, m); 3H à 0,99 ppm ($H_f$, d, $J_{fe}$=7); 3H à 0,95 ppm ($H_h$, t, $J_{hg}$=7) |
| 4 b | CH e<br>$(CH_3)_2$ f | 93 | −19 | 1H à 9,66 ppm ($H_a$, S); 1H à 5,33 ppm ($H_c$, d, $J_{cb}$=7); 1H à 4,23 ppm ($H_b$, dd, $J_{bc}$=7); 1H à 2,29 ppm ($H_e$, m); 9H à 1,46 ppm ($H_d$, S); 6H à 0,99 ppm ($H_f$, 2d, $J_{fe}$=7) |
| 5 b | 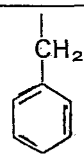 | 86 | −44,4 | F=86 |
| 6 b | 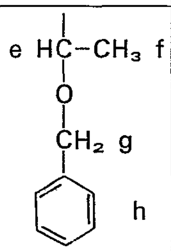 | 95 | +16 | 1H à 9,60 ppm ($H_a$, S); 5H à 7,31 ppm ($H_h$, m); 1H à 5,40 ppm ($H_c$, d, $J_{cb}$=7,5); 1H à 4,57 ppm ($H_g$, d, $J_{gg'}$=11,5); 1H à 4,40 ppm ($H_{g'}$, d, $J_{g'g}$=11,5); 2H à 4,25 ppm ($H_b$, $H_e$, m); 9H à 1,46 ppm ($H_d$, S); 3H à 1,25 ppm ($H_f$, d, $J_{fe}$=6,5) |

Note 1 : Par suite de la faible solubilité de l'amide de départ, on effectue ici la réduction dans le tétrahydrofuranne au lieu de l'éther.

Les α-alkyl aminoaldéhydes ainsi préparés sont en particulier des intermédiaires de synthèse intéressants pour la préparation de composés peptidiques doués de propriétés thérapeutiques.

**Revendications**

1. Procédé de préparation d'α-alkylaminoaldéhydes de formule

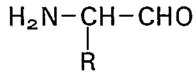

dans laquelle R est un groupe alkyle droit ou ramifié ou un groupe aralkyle pouvant être substitué par au moins un groupe hydroxy, thiol ou NHR', R' étant H ou un groupe alkyle, caractérisé en ce que:

a) on utilise comme produit de départ un ester activé de l'aminoacide de formule:

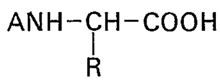

dans laquelle les fonctions amines ont été bloquées de façon réversible par un radical tel que A,

b) on fait réagir sur ce produit de départ la N,O-diméthylhydroxylamine en milieu basique pour obtenir un produit de formule:

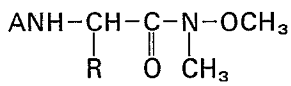

c) puis on réduit le produit ainsi obtenu par action d'un hydrure tel que l'hydrure double de lithium-aluminium, et

d) enfin, si nécessaire, on élimine le ou les groupes utilisés pour le blocage.

2. Procédé selon la revendication 1, caractérisé en ce que l'étape b est réalisée en solution dans un solvant convenable tel que le chlorure de méthylène ou l'acétate d'éthyle.

3. Procédé selon l'une des revendications 1 ou 2, caractérisé en ce que l'étape d est effectuée à basse température, de préférence aux environs de 0° C; dans un solvant tel que l'éther ou le tétrahydrofuranne.

## Patentansprüche

1. Verfahren zur Herstellung von α-Alkylaminoaldehyden der Formel

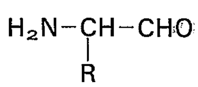

worin R eine gerade oder verzweigte Alkylgruppe oder eine ggf. durch mindestens eine Hydroxy-, thiol- oder NHR'-Gruppe, worin R' für H oder eine Alkylgruppe steht, substituierte Aralkylgruppe darstellt, dadurch gekennzeichnet, dass

a) als Ausgangsprodukt ein aktivierter Aminosäureester der Formel

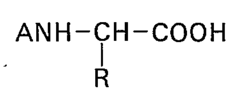

worin die Aminfunktionen durch ein Radikal A reversibel geschützt wurden, verwendet wird,

b) dieses Ausgangsprodukt mit N,O-Dimethylhydroxylamin in basischem Milieu zur Umsetzung gebracht wird, um, ein Produkt der Formel

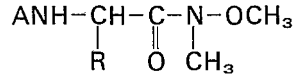

zu erhalten,

c) dann das so erhaltenen Produkt durch Einwirken eines Hydrids, wie Lithium-Aluminiumhydrid, reduziert wird, und

d) schliesslich notwendigfalls die zum Schutz verwendete(n) Gruppe(n) entfernt wird (werden).

2. Verfahren nach Anspruch 1, dadurch gekennzeichnet, dass die Stufe b in Lösung in einem geeigneten Lösungsmittel, wie Methylenchlorid oder Äthylacetat, durchgeführt wird.

3. Verfahren nach einem der Ansprüche 1 oder 2, dadurch gekennzeichnet, dass die Stufe d bei niedriger Temperatur, vorzugsweise etwa 0° C, in einem Lösungsmittel, wie Äther oder Tetrahydrofuran, durchgeführt wird.

## Claims

1. Process for preparing α-alkylaminoaldehydes of formula:

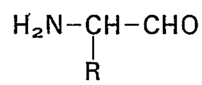

in which R is a straight or branched alkyl group or an aralkyl group which may be substituted by at least one hydroxy, thiol or NHR' group, R' being H or an alkyl group, characterized in that it comprises the steps of:

(*a*) using as starting product an activated ester of the amino acid of formula:

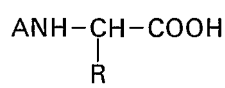

in which the amine functions have been reversibly blocked by a radical such as A,

(*b*) reacting on this starting product N,O-dimethylhydroxylamine in a basic medium to obtain a product of formula:

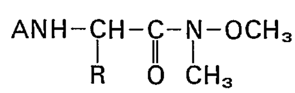

(*c*) then reducing the product thus obtained by action of a hydride such as the double hydride of lithium-aluminium, and

(*d*) finally, if necessary, eliminating the or each group used for blocking.

2. The process according to Claim 1, characterized in that step (*b*) is carried out in solution in an appropriate solvent such as methylenechloride or ethylacetate.

3. The process according to one of Claims 1 or 2, characterized in that step (*d*) is effected at low temperature, preferably at about 0° C, in a solvent such as ether or tetrahydrofuran.